# EUROPÄISCHE PATENTSCHRIFT

(11) **EP 2 951 163 B1**
(45) Veröffentlichungstag und Bekanntmachung des Hinweises auf die Patenterteilung: **07.09.2016**
(21) Anmeldenummer: 14701583.8
(22) Anmeldetag: 29.01.2014
(51) Int. Cl.: C07D 251/24, G03C 1/815

(54) **VERFAHREN ZUR HERSTELLUNG EINES POLYMERISIERBAREN UV-ABSORBERS**
METHOD FOR PRODUCING A POLYMERIZABLE UV ABSORBER
PROCÉDÉ DE FABRICATION D'UN ABSORBEUR À UV POLYMÉRISABLE

(30) Priorität: 01.02.2013 EP 13153704; 24.04.2013 EP 13165168
(43) Veröffentlichungstag der Anmeldung: 09.12.2015
(73) Patentinhaber: Covestro Deutschland AG, 51373 Leverkusen (DE)
(72) Erfinder: KOSTROMINE, Serguei, 53913 Swisttal-Buschhoven (DE); KÜHN, Frauke, 51373 Leverkusen (DE)
(74) Vertreter: Levpat
(86) Internationale Anmeldenummer: PCT/EP2014/051722
(87) Internationale Veröffentlichungsnummer: WO 2014/118233

(56) Entgegenhaltungen:
- EP-A1- 0 706 083
- EP-A1- 1 094 094
- GB-A- 2 293 823

## Beschreibung

Die vorliegende Erfindung betrifft das Herstellungsverfahren eines organischen UV-Absorbers, der eine (Meth)acrylat-Gruppe im Molekül aufweist und dadurch für härtbare UV-Schutzbeschichtungen besonders geeignet ist.

Für Außenanwendungen müssen transparente Kunststoffartikel, wie z.B. Platten, Folien, Spritzgussbauteile oder Extrusionsformkörper, vor allem mittels UV-Schutz vor der aggressiven Sonnenstrahlung und mittels Kratzfestausrüstung vor mechanischen Einwirkungen geschützt werden. Ein gängiges Verfahren dafür ist die obere und manchmal auch die einzige Schutzschicht, die kratzfest sein muss, zusätzlich mit UV-Schutzfunktion zu versehen und dafür mit einer wesentlichen Menge von UV-Absorbern zu bestücken (vgl. DE-A 10 2006 016 642). Die klassischen UV-Absorber wirken allerdings in den Schutzschichten als Weichmacher und verringern die mechanische Beständigkeit der Schicht.

Der Einfluss von UV-Absorbern auf die mechanische und chemische Beständigkeit der Beschichtung ist umso größer, je höher seine Konzentration in der Beschichtung ist. Man braucht aber eine gewisse Konzentration des Absorbers, um UV-Licht effektiv zu absorbieren und das Substrat sicher und dauerhaft davor zu schützen. Diese Konzentration wird laut Lambert-Beer-Gesetz umso höher sein, je dünner die Beschichtung ist. Für die modernen Beschichtungen, deren Schichtstärke zwischen 1 und 10 µm liegen könnte, bedeutet dies, dass sie bis zu 10 Gew.-% des UV-Absorbers beinhalten sollten, um die notwendige Absorptionskraft aufzuweisen. Die mechanische und chemische Beständigkeit solcher Beschichtungen wird dadurch negativ beeinträchtigt.

Eine Lösung des Problems könnte es sein, wenn der UV-Absorber nicht als passives Additiv vorliegt, sondern in dem Härtungsprozess chemisch aktiv teilnimmt und sich ins Polymergerüst der Beschichtung einbindet.

Nicht nur die Härte wird dadurch positiv beeinflusst. Der chemisch gebundene UV-Absorber kann nicht zur äußeren Oberfläche der Beschichtung migrieren und im Laufe der Bewitterung abgewaschen/abgetragen werden. Die Schutzkraft der Beschichtung bleibt dadurch also erhalten.

Eine der besten UV-Absorber für Polycarbonat sind Biphenyl-substituierte Triazine (vgl. WO-A 2006/108520). Diese Substanzklasse zeigt eine hervorragende Absorptionswirkung bei 320 - 380 nm und gleichzeitig eine sehr hohe eigene UV-Stabilität (WO 2000/066675 A1, US-A 6,225,384, US 5,869,588).

Um an dem chemischen Netzwerk der Beschichtung angebunden zu sein, sollten solche Moleküle mit polymerisierbaren Acrylat- und/oder Methacrylat-Gruppen ausgestattet werden.

Beispiele solcher Biphenyl-substituierten Triazine sind bekannt (EP 2 447 236 A1, WO 2011/040541 A1). Die letztgenannte internationale Patentanmeldung offenbart beispielsweise:

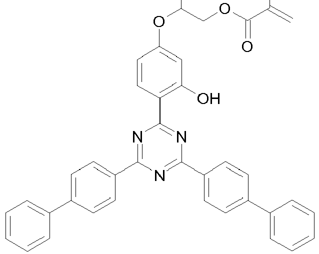

Wünschenswert wären in diesem Zusammenhang einfache Herstellungsverfahren für Vertreter der obigen Strukturen, welche von gut verfügbaren Einsatzstoffen ausgehen. Die vorliegende Erfindung hat sich die Aufgabe gestellt, ein solches Herstellungsverfahren bereitzustellen.

Erfindungsgemäß wurde die Aufgabe gelöst durch ein Verfahren zur Herstellung von Verbindungen der allgemeinen Formel (I)

A-X-CH₂-O-C(=O)-C(R)=CH₂ (I),

wobei
A für 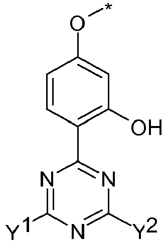 steht, worin
Y¹ und Y² unabhängig voneinander für Substituenten der generellen Formel 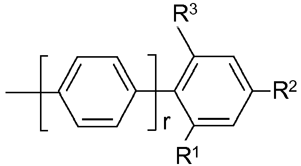 stehen, worin
r für 0 oder 1, bevorzugt für 1 steht,
R¹, R², R³ unabhängig voneinander für H, OH, C₁₋₂₀-Alkyl, C₄₋₁₂-Cycloalkyl, C₂₋₂₀-Alkenyl, C₁₋₂o-Alkoxy, C₄₋₁₂-Cycloalkoxy, C₂₋₂₀-Alkenyloxy, C₇₋₂₀-Aralkyl, Halogen, -C=N, C₁₋₅-Haloalkyl,-SO₂R', -SO₃H, -SO₃M (M = Alkalimetall), -COOR', -CONHR', -CONR'R", -OCOOR', -OCOR',-OCONHR', (Meth)acrylamino, (Meth)acryloxy, gegebenenfalls durch C₁₋₁₂-Alkyl, C₁₋₁₂-Alkoxy, CN und/oder Halogen substituiertes C₆₋₁₂-Aryl oder gegebenenfalls durch C₁₋₁₂-Alkyl, C₁₋₁₂-Alkoxy, CN und/oder Halogen substituiertes C₃₋₁₂-Heteroaryl stehen, worin
M für ein Alkalimetallkation steht,
R' und R" für H, C₁₋₂₀-Alkyl, C₄₋₁₂-Cycloalkyl, gegebenenfalls durch C₁₋₁₂-Alkyl, C₁₋₁₂-Alkoxy, CN und/oder Halogen substituiertes C₆₋₁₂-Aryl oder gegebenenfalls durch C₁₋₁₂-Alkyl, C₁₋₁₂-Alkoxy, CN und/oder Halogen substituiertes C₃₋₁₂-Heteroaryl stehen,
X für verzweigtes oder unverzweigtes C₁₋₂₀-Alkyl steht und
R für H oder CH₃ steht.

Das erfindungsgemäße Verfahren umfasst die folgenden Schritte:
Reduktion einer Verbindung der allgemeinen Formel (II)

   A - X-C(=O)-O-R"' (II)

   wobei A und X die obigen Bedeutungen aufweisen und R'" für verzweigtes oder unverzweigtes C₁₋₂₀-Alkyl, C₄₋₁₂-Cycloalkyl oder gegebenenfalls durch C₁₋₁₂-Alkyl, C₁₋₁₂-Alkoxy, CN und/oder Halogen substituiertes C₆₋₁₂-Aryl steht;
zu einer Verbindung der allgemeinen Formel (IIa)

   A - X-CH₂OH (IIa)

Umsetzung der zuvor erhaltenen Verbindung (IIa) mit (Meth)Acrylsäure und/oder einem Anhydrid, Säurechlorid oder Ester der (Meth)Acrylsäure.

Der erste Schritt des Herstellungsverfahrens ist eine Reduktion der Ester-Gruppe von Ausgangsverbindungen (I) in eine -CH₂-OH-Gruppe. Hier kann die ganze Palette der Reduktionsmittel eingesetzt werden, die dafür bekannt sind, eine aliphatische Ester-Gruppe bis zu einem Alkohol zu reduzieren (C. Ferri, Reaktionen der organischen Synthese, Georg Thieme Verlag, Stuttgart, 1978, S.865).

Die verwendeten Lösemittel sind spezifisch für jedes Reduktionsmittel und dem Fachmann bekannt. Für Aluminiumhydride sind dies z.B. Ether, THF, Alkane, Cycloalkane, Toluol, Pyridin und N-Alkyl-morpholin. Boranate, wie Natriumboranat werden üblicherweise in Wasser, wässrigem Alkohol, Isopropylalkohol, Acetonitril angewendet.

Theoretisch braucht man für die Reduktion von 1 mol eines Esters in einen Alkohol 0,5 mol Reduktionsmittel wie Natriumboranat. In der Praxis arbeitet man mit einem Überschuss von Natriumboranat. Erfindungsgemäß nimmt man für 1 mol der Ausgangsverbindung in der Regel zwischen 0,6 und 3 mol Reduktionsmittel, vorteilhaft zwischen 0,75 und 2,5 mol Reduktionsmittel.

Nach der Reaktion soll der Überschuss des Reduktionsmittels sicher desaktiviert werden, bevor man beginnt, Produkt-Intermediate mit Säure zu zerstören. Den Überschuss des Reduktionsmittels desaktiviert man im Fall der Erfindung durch Zugabe von einfachen aliphatischen oder cycloaliphatischen Ketonen, die mit dem überschüssigen Reduktionsmittel sanft abreagieren. Bevorzugt ist dabei Aceton, das sich dadurch in i-Propanol umwandelt.

Der zweite Schritt des Herstellungsverfahrens ist ein (Meth)acrylieren der gewonnenen OH-Gruppe des UV-Absorbers. Der Aufbau des (Meth)acrylsäureesters der Zielverbindung geht erfindungsgemäß durch Reaktion des zuvor erhaltenen Alkohols mit (Meth)acrylsäure oder mit einem Anhydrid, Chlorid oder Ester der (Meth)acrylsäure. Vorzugsweise nimmt man dafür das Chlorid oder einen Ester der (Meth)acrylsäure. Besonders praktikabel in großem Maßstab, kosteneffizient und dadurch besonders bevorzugt ist die Umesterung des Alkohols mit einem (Meth)acrylsäureester.

Unter den (Meth)acrylsäureestern sind einfache aliphatische Ester des Methacrylsäure bevorzugt, wie Methyl-, Ethyl-, Propyl- oder Butyl(meth)acrylat. Besonders bevorzugt dabei ist Methyl(meth)acrylat.

Zur Katalyse dieser Umesterung können Säuren, Basen oder metallorganische Verbindungen eingesetzt werden. Keiner der bekannten Katalysatoren ist dabei grundsätzlich ausgeschlossen. Vorteilhaft sind aber die Säuren und insbesondere wasserlösliche Säuren, die aus dem Rohprodukt leicht mit Wasser abgewaschen werden können. Besonders vorteilhaft sind Benzol- und p-Toluolsulfonsäuren. Pro mol des Zwischenprodukts (IIa) verwendet man beispielsweise von 0,5 bis 1,5 mol des Säurekatalysators.

Ein Lösemittel ist bei der Umesterung nicht immer erforderlich. Der im Überschuss eingesetzte (Meth)acrylsäureester kann als Lösemittel für das Ausgangsmaterial und Produkt dienen. Pro mol der Substanz (IIa) nimmt man beispielsweise 30 bis 45 mol Methacrylsäureester, das am Ende der Reaktion aus dem Reaktionsgemisch teilweise wiedergewonnen werden kann.

Alternativ zu der besonders vorteilhaften Umesterung ist die vorteilhafte Umsetzung der Substanz der Formel (IIa) mit Methacrylsäurechlorid. Das molare Verhältnis der beiden Reaktanden liegt in der Regel zwischen 1: 1,1 und 1:1,3. Als HCl-Fänger kann man tertiäre Amine oder Pyridin verwenden. Vorteilhaft ist Triethylamin. Das molare Verhältnis zwischen der Substanz der Formel (IIa) und Triethylamin liegt zwischen 1: 1,1 und 1:1,5. Als Lösemittel nutzt man beispielsweise cyclische Ether. Vorteilhaft ist Dioxan.

In der Ausgangsverbindung (II) werden für R'" vorzugsweise einfache Alkylreste wie Methyl, Ethyl, Propyl, Butyl, Pentyl, Hexyl, Heptyl oder Octyl ausgewählt. Besonders geeignet sind n-Octyl und iso-Octyl.

Ausführungsformen und weitere Aspekte der vorliegenden Erfindung werden nachfolgend näher beschrieben. Sie können beliebig miteinander kombiniert werden, sofern sich aus dem Kontext nicht eindeutig das Gegenteil ergibt.

In einer Ausführungsform des erfindungsgemäßen Verfahrens steht X für CH(CH₃).

In einer weiteren Ausführungsform des erfindungsgemäßen Verfahrens steht in den Substituenten Y¹ und Y² jeweils r für 1.

In einer weiteren Ausführungsform des erfindungsgemäßen Verfahrens stehen in den Substituenten Y¹ und Y² jeweils die Reste R¹, R² und R³ für H.

Bevorzugt werden für die Ausgangsverbindung (II) solche der allgemeinen Formel (II-1) ausgewählt: 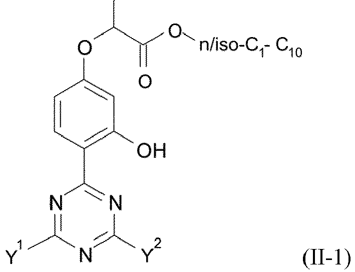
worin Y¹ und Y² die vorangehend für die Verbindungen der allgemeinen Formel (I) genannte Bedeutung haben.
Besonders bevorzugt stehen die Substituenten Y¹ und Y² in den Formeln (II) und (II-1) gleichzeitig für

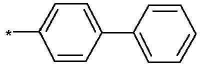

Ganz besonders bevorzugt ist die folgende Verbindung als Ausgangsverbindung (II), welche unter dem Handelsnamen Tinuvin® 479 erhältlich ist:

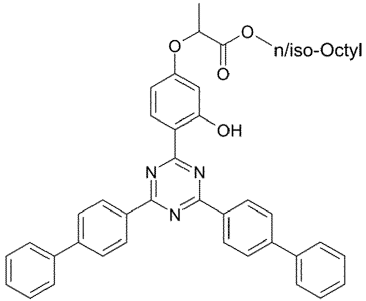

Diese Verbindung (propanoic acid,2-[4-[4,6-bis([1,1'-biphenyl]-4-yl)-1,3,5-triazin-2-yl]-3-hydroxyphenoxy]-,n-/iso-octyl ester) ist kommerziell erhältlich und besitzt die CAS-Nummer 204848-45-3.

In einer weiteren Ausführungsform des erfindungsgemäßen Verfahrens erfolgt die Reduktion der Verbindung (II) zur Verbindung (IIa) mit einem komplexen Hydrid als Reduktionsmittel. Vorteilhaft dabei sind Hydride wie Aluminiumhydride, Boranate wie Natriumboranat oder Borane bzw. Borankomplexe mit Ethern und/oder Lewis-Säuren. Besonders vorteilhaft ist Natriumboranat.

In einer weiteren Ausführungsform des erfindungsgemäßen Verfahrens wird die Reduktion der Verbindung (II) zur Verbindung (IIa) in einem Lösungsmittelgemisch umfassend Tetrahydrofuran und einem Alkohol durchgeführt. Überraschenderweise wurde festgestellt, dass beispielsweise die erfindungsgemäße Reduktion von Tinuvin 479 vorteilhaft mit Natriumboranat in einer Lösungsmittelmischung aus THF und einem Alkohol, wie Methanol, Ethanol, n- und i-Propanol, 1-Methoxy-2-propanol, n-, i- und t-Butanol verläuft. Besonders vorteilhaft dabei ist i-Propanol.

Das Verhältnis zwischen THF und einem Alkohol, wie i-Propanol, in der Mischung liegt im Intervall zwischen 1:9 und 9:1, vorteilhaft zwischen 3:7 und 7:3, besonders vorteilhaft zwischen 4:6 und 6:4 bezogen auf das Gewicht.

In einer weiteren Ausführungsform des erfindungsgemäßen Verfahrens erfolgt die Umsetzung der Verbindung (IIa) mit (Meth)Acrylsäure und/oder (Meth)Acrylsäureestern und in Gegenwart von Benzolsulfonsäure und/oder Toluolsulfonsäure als Katalysator.

### Beispiele

Die Erfindung wird anhand der nachfolgenden Beispiele weiter beschrieben, ohne jedoch darauf beschränkt zu sein.

### Beispiel 1

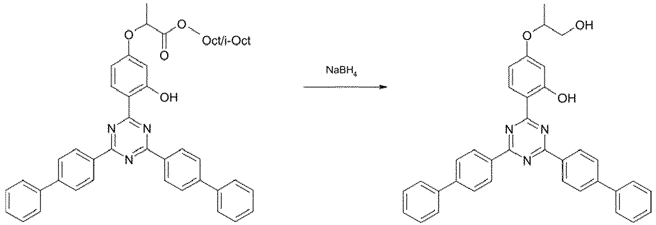

17,9 g Tinuvin® 479 (BASF) wurden in 18 g wasserfreiem THF bei RT (Raumtemperatur) gelöst und 18 g 2-Propanol wurden hinzugegeben. 2,57 g Natriumborhydrid wurden hinzugesetzt (exotherm bis ca. 50°C) und das Reaktionsgemisch wurde bei 72 °C für 5 Stunden nachgerührt (nach ca. 2 h sichtbare Bildung eines Niederschlags, nach 4,5 h Nachrührzeit Zusatz von 18 ml Tetrahydrofuran und 18 ml Isopropanol). Der Reaktionsverlauf wurde per Dünnschichtchromatographie mit Toluol/Ethylacetat = 4: 1 kontrolliert. Anschließend wurde die Reaktionsmischung abgekühlt und mit 15,34 g Aceton tropfenweise versetzt (unter Kühlung mit Eisbad) und anschließend noch 45 min. nachgerührt. Danach wurden unter Kühlung im Eisbad 12,6 ml IN Salzsäure tropfenweise zugegeben und es wurde gerührt, bis keine Gasbildung mehr auftrat. 120 ml Methanol wurden dann zugegeben und man rührte für 30 min. Der erhaltene Feststoff wurde abfiltriert und auf dem Filter mit 50 ml Methanol nachgewaschen. Anschließend wurde der Feststoff in 120 ml Wasser aufgeschlämmt und für 1 h gerührt. Der Feststoff wurde abfiltriert (Nutsche Durchmesser: 10 cm), mit 50 ml Wasser und 50 ml Methanol nachgespült und im Vakuum bei 40 °C getrocknet Das Produkt wurde nochmals mit Dünnschichtchromatographie (Toluol /Ethylacetat = 4: 1) kontrolliert.
Ausbeute: 10,4 g (71,4 % d.Th.)
Schmelzpunkt: 206°C
Elementaranalyse: C₃₆H₂₉N₃O₃ (551,65); Ber.: C78,38; H5,30; N7,62; Gef.: C78,10; H5,30; N7,40.

### Beispiel 2

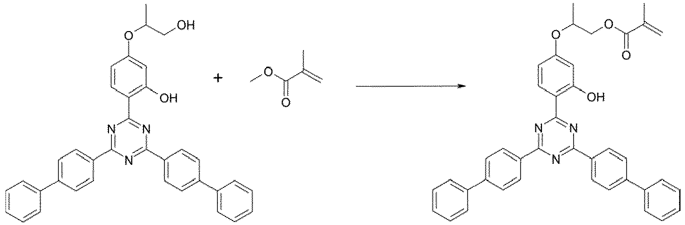

10 g eines Produktes gemäß Beispiel 1, 70 g Methacrylsäuremethylester, 5 g p-Toluolsulfonsäure Monohydrat und 0,2 g Methoxyphenol wurden auf einmal zusammen in einer Destillationsapparatur vorgelegt. Beim Rühren stellte man das Ölbad auf 120°C. Die Destillation begann bei 60 °C (Kopf) und wurde bei 86 °C beendet. Man destillierte solange, bis 45g Destillat übergegangen waren, nicht darüber hinaus. Dies dauerte ca. 2h. Das Reaktionsgemisch wurde auf 40-50 °C abgekühlt. 100 ml Methanol wurden der Reaktionsmischung bei kräftigem Rühren auf einmal zuzugeben. Es wurde eine halbe Stunde nachgerührt. Die erhaltenen Kristalle wurden abgesaugt, mit Methanol auf dem Filter gewaschen und im Vakuum getrocknet.
Ausbeute: 12,3 g.

Die Kristalle löste man in 50 ml einer Mischung aus Toluol/Ethylacetat=32:1 und filtrierte durch eine 1 cm dicke Schicht Kieselgel. Es wurde mit 300 ml einer Mischung Toluol/Ethylacetat = 32:1 nachgespült. Methoxyphenol (0,02 g) wurde der Lösung als Stabilisator zuzugeben. Das Lösemittel wurde abgedampft. Methanol wurde erneut zugegeben. Die Kristalle wurden abfiltriert und im Vakuum getrocknet
Ausbeute: 6,9 g (61% d. Th.)
Schmelzpunkt: 88 °C
Elementaranalyse: C₄₀H₃₃N₃O₄ (619,73); Ber.: C77,53; H5,37; N6,78; Gef.: C77,20; H5,80; N6,40.

### Beispiel 2a

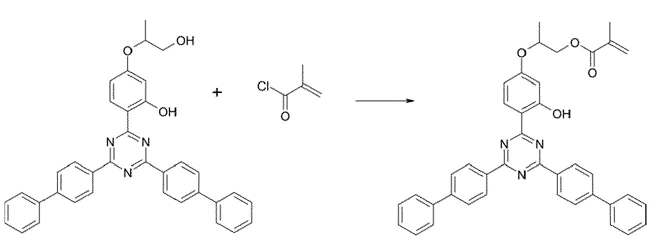

160 g eines Produkts gemäß Beispiel 1 wurden in 1100 ml Dioxan bei 70 °C zum größten Teil gelöst. 45 g Triethylamin wurden zugegeben. Das Reaktionsgemisch wurde auf Raumtemperatur abgekühlt. Eine Lösung aus 33,3 g Methacrylsäurechlorid und 160ml Dioxan wurde dem Reaktionsgemisch tropfenweise zugegeben. Das Reaktionsgemisch wurde noch 4 Stunden bei Raumtemperatur gerührt und danach in 3 L Wasser eingegossen. Der Niederschlag wurde abfiltriert, getrocknet und in eine Mischung aus Toluol und Ethylacetat (8:1) gegeben. Der ungelöste Teil (ca. 15 g, bestehend aus Ausgangsmaterial der Formel 2) wurde abfiltriert, getrocknet und später für die gleiche Synthese noch einmal verwendet. Die Lösung wurde durch eine 10 cm Kieselgel-Schicht filtriert. Methoxyphenol (0,02 g) wurde der filtrierten Lösung als Stabilisator zuzugeben. Das Lösemittel wurde abgedampft. Methanol wurde dem Rest zugegeben. Kristalle der Zielverbindung wurden abfiltriert und im Vakuum getrocknet.
Ausbeute: 100 g (56% d. Th.)

## Patentansprüche

1. Verfahren zur Herstellung von Verbindungen der allgemeinen Formel (I)
A-X-CH₂-O-C(=O)-C(R)=CH₂ (I),
wobei
A für 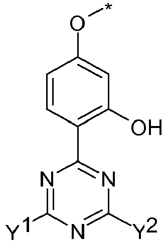 steht, worin
Y¹ und Y² unabhängig voneinander für Substituenten der generellen Formel 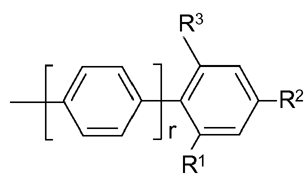 stehen, worin
r für 0 oder 1, bevorzugt für 1 steht,
R¹, R², R³ unabhängig voneinander für H, OH, C₁₋₂₀-Alkyl, C₄₋₁₂-Cycloalkyl, C₂₋₂₀-Alkenyl, C₁₋₂₀-Alkoxy, C₄₋₁₂-Cycloalkoxy, C₂₋₂₀-Alkenyloxy, C₇₋₂₀-Aralkyl, Halogen, -C=N, C₁₋₅-Haloalkyl, -SO₂R', -SO₃H,-SO₃M (M = Alkalimetall), -COOR', -CONHR', -CONR'R",-OCOOR', -OCOR', -OCONHR', (Meth)acrylamino, (Meth)acryloxy, gegebenenfalls durch C₁₋₁₂-Alkyl, C₁₋₁₂-Alkoxy, CN und/oder Halogen substituiertes C₆₋₁₂-Aryl oder gegebenenfalls durch C₁₋₁₂-Alkyl, C₁₋₁₂-Alkoxy, CN und/oder Halogen substituiertes C₃₋₁₂-Heteroaryl stehen, worin
M für ein Alkalimetallkation steht,
R' und R" für H, C₁₋₂₀-Alkyl, C₄₋₁₂-Cycloalkyl, gegebenenfalls durch C₁₋₁₂-Alkyl, C₁₋₁₂-Alkoxy, CN und/oder Halogen substituiertes C₆₋₁₂-Aryl oder gegebenenfalls durch C₁₋₁₂-Alkyl, C₁₋₁₂-Alkoxy, CN und/oder Halogen substituiertes C₃₋₁₂-Heteroaryl stehen,
X für verzweigtes oder unverzweigtes C₁₋₂₀-Alkyl steht,
R für H oder CH₃ steht,
**dadurch gekennzeichnet, dass** das Verfahren die folgenden Schritte umfasst:
- Reduktion einer Verbindung der allgemeinen Formel (II)
A-X-C(=O)-O-R"' (II)
wobei A und X die obigen Bedeutungen aufweisen und R'" für verzweigtes oder unverzweigtes C₁₋₂₀-Alkyl, C₄₋₁₂-Cycloalkyl oder gegebenenfalls durch C₁₋₁₂-Alkyl, C₁₋₁₂-Alkoxy, CN und/oder Halogen substituiertes C₆₋₁₂-Aryl steht;
zu einer Verbindung der allgemeinen Formel (IIa)
A-X-CH₂OH (IIa)
- Umsetzung der zuvor erhaltenen Verbindung (IIa) mit (Meth)Acrylsäure und/oder einem Anhydrid, Säurechlorid oder Ester der (Meth)Acrylsäure.

2. Verfahren gemäß Anspruch 1, **dadurch gekennzeichnet, dass** X für CH(CH₃) steht.

3. Verfahren gemäß Anspruch 1 oder 2, **dadurch gekennzeichnet, dass** in den Substituenten Y¹ und Y² jeweils r für 1 steht.

4. Verfahren gemäß wenigstens einem der Ansprüche 1 bis 3, **dadurch gekennzeichnet, dass** in den Substituenten Y¹ und Y² jeweils die Reste R¹, R² und R³ für H stehen.

5. Verfahren gemäß wenigstens einem der Ansprüche 1 bis 4, **dadurch gekennzeichnet, dass** die Reduktion der Verbindung (II) zur Verbindung (IIa) mit einem komplexen Hydrid als Reduktionsmittel erfolgt.

6. Verfahren gemäß wenigstens einem der Ansprüche 1 bis 5, **dadurch gekennzeichnet, dass** die Reduktion der Verbindung (II) zur Verbindung (IIa) in einem Lösungsmittelgemisch umfassend Tetrahydrofuran und einem Alkohol durchgeführt wird.

7. Verfahren gemäß wenigstens einem der Ansprüche 1 bis 6, **dadurch gekennzeichnet, dass** die Umsetzung der Verbindung (IIa) mit (Meth)Acrylsäure und/oder (Meth)Acrylsäureestern und in Gegenwart von Benzolsulfonsäure und/oder Toluolsulfonsäure als Katalysator erfolgt.

## Claims

1. Propcess for preparing compounds of the general formula (I)
A-X-CH₂-O-C(=O)-C(R)=CH₂ (I),
where
A is 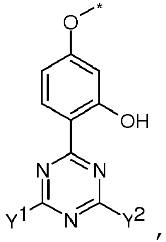 in which
Y¹ and Y² independently of one another are substituents of the general formula 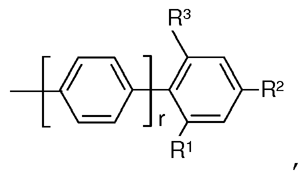 in which
r is 0 or 1, preferably 1,
R¹, R², and R³ independently of one another are H, OH, C₁₋₂₀-alkyl, C₄₋₁₂-cycloalkyl, C₂₋₂₀-alkenyl, C₁₋₂₀-alkoxy, C₄₋₁₂-cycloalkoxy, C₂₋₂₀-alkenyloxy, C₇₋₂₀-aralkyl, halogen,-C≡N, C₁₋₅-haloalkyl, -SO₂R', -SO₃H,-SO₃M (M = alkali metal), -COOR',-CONHR', -CONR'R", -OCOOR', -OCOR', -OCONHR', (meth)acrylamino, (meth)acryloyloxy, C₆₋₁₂-aryl substituted optionally by C₁₋₁₂-alkyl, C₁₋₁₂-alkoxy, CN and/or halogen, or C₃₋₁₂-heteroaryl substituted optionally by C₁₋₁₂-alkyl, C₁₋₁₂-alkoxy, CN and/or halogen, and in which
M is an alkali metal cation,
R' and R" are H, C₁₋₂₀-alkyl, C₄₋₁₂-cycloalkyl, C₆₋₁₂-aryl substituted optionally by C₁₋₁₂-alkyl, C₁₋₁₂-alkoxy, CN and/or halogen or C₃₋₁₂-heteroaryl substituted optionally by C₁₋₁₂-alkyl, C₁₋₁₂-alkoxy, CN and/or halogen,
X is branched or unbranched C₁₋₂₀-alkyl,
R is H or CH₃,
**characterized in that** the process comprises the following steps:
- reducing a compound of the general formula (II)
A-X-C(=O)-O-R''' (II)
where A and X have the above definitions and R''' is branched or unbranched C₁₋₂₀-alkyl, C₄₋₁₂-cycloalkyl, or C₆₋₁₂-aryl substituted optionally by C₁₋₁₂-alkyl, C₁₋₁₂-alkoxy, CN and/or halogen;
to give a compound of the general formula (IIa)
A-X-CH₂OH (IIa)
- reacting the compound (IIa) obtained above with (meth)acrylic acid and/or with an anhydride, acid chloride or ester of (meth)acrylic acid.

2. Process according to Claim 1, **characterized in that** X is CH(CH₃).

3. Process according to Claim 1 or 2, **characterized in that** r in each of the substituents Y¹ and Y² is 1.

4. Process according to at least one of Claims 1 to 3, **characterized in that** the radicals R¹, R² and R³ in each of the substituents Y¹ and Y² are H.

5. Process according to at least one of Claims 1 to 4, **characterized in that** the reducing of the compound (II) to the compound (IIa) takes place with a complex hydride as reducing agent.

6. Process according to at least one of Claims 1 to 5, **characterized in that** the reducing of the compound (II) to the compound (IIa) is carried in a solvent mixture encompassing tetrahydrofuran and an alcohol.

7. Process according to at least one of Claims 1 to 6, **characterized in that** the reacting of the compound (IIa) takes place with (meth)acrylic acid and/or (meth)acrylic esters and in the presence of benzenesulfonic acid and/or toluenesulfonic acid as catalyst.

## Revendications

1. Procédé pour la préparation de composés de formule générale (I)
A-X-CH₂-O-C(=O)-C(R)=CH₂ (I),
dans laquelle
A représente 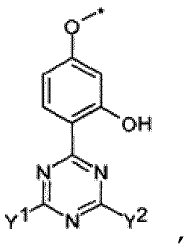 où
Y¹ et Y² représentent, indépendamment l'un de l'autre, des substituants de formule générale 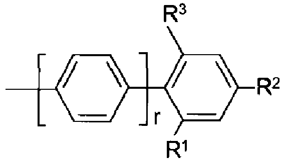 dans laquelle
r représente 0 ou 1, de préférence 1,
R¹, R², R³ représentent, indépendamment les uns des autres, H, OH, C₁₋₂₀-alkyle, C₄₋₁₂-cycloalkyle, C₂₋₂₀-alcényle, C₁₋₂₀-alcoxy, C₄₋₁₂-cycloalcoxy, C₂₋₂₀-alcényloxy, C₇₋₂₀-aralkyle, halogène, -C=N, C₁₋₅-halogénoalkyle, -SO₂R', -SO₃H, -SO₃M (M = métal alcalin), -COOR', -CONHR', -CONR'R", -OCOOR', -OCOR', -OCONHR', (méth)acrylamino, (méth)acryloxy ; C₆₋₁₂-aryle le cas échéant substitué par C₁₋₁₂-alkyle, C₁₋₁₂-alcoxy, CN et/ou halogène ; ou C₃₋₁₂-hétéroaryle le cas échéant substitué par C₁₋₁₂-alkyle, C₁₋₁₂-alcoxy, CN et/ou halogène, où
M représente un cation de métal alcalin,
R' et R" représentent H, C₁₋₂₀-alkyle, C₄₋₁₂-cycloalkyle ; C₆₋₁₂-aryle le cas échéant substitué par C₁₋₁₂-alkyle, C₁₋₁₂-alcoxy, CN et/ou halogène ; ou C₃₋₁₂-hétéroaryle le cas échéant substitué par C₁₋₁₂-alkyle, C₁₋₁₂-alcoxy, CN et/ou halogène,
X représente C₁₋₂₀-alkyle ramifié ou non ramifié,
R représente H ou CH₃,
**caractérisé en ce que** le procédé comprend les étapes suivantes, consistant à :
- réduire un composé de formule générale (II)
A-X-C(-O)-O-R"' (II)
A et X présentant les significations ci-dessus et R"' représentant C₁₋₂₀-alkyle ramifié ou non ramifié, C₄₋₁₂-cycloalkyle ; ou C₆₋₁₂-aryle le cas échéant substitué par C₁₋₁₂-alkyle, C₁₋₁₂-alcoxy, CN et/ou halogène ;
en un composé de formule générale (IIa)
A-X-CH₂OH (IIa)
- transformer le composé (IIa) obtenu ci-dessus avec de l'acide (méth)acrylique et/ou un anhydride, un chlorure d'acide ou un ester de l'acide (méth)acrylique.

2. Procédé selon la revendication 1, **caractérisé en ce que** X représente CH(CH₃).

3. Procédé selon la revendication 1 ou 2, **caractérisé en ce que**, dans les substituants Y¹ et Y², r représente à chaque fois 1.

4. Procédé selon au moins l'une quelconque des revendications 1 à 3, **caractérisé en ce que**, dans les substituants Y¹ et Y², les radicaux R¹, R² et R³ représentent à chaque fois H.

5. Procédé selon au moins l'une quelconque des revendications 1 à 4, **caractérisé en ce que** la réduction du composé (II) en composé (IIa) a lieu avec un hydrure complexe comme agent de réduction.

6. Procédé selon au moins l'une quelconque des revendications 1 à 5, **caractérisé en ce que** la réduction du composé (II) en composé (IIa) est réalisée dans un mélange de solvants comprenant du tétrahydrofuranne et un alcool.

7. Procédé selon au moins l'une quelconque des revendications 1 à 6, **caractérisé en ce que** la transformation du composé (IIa) a lieu avec de l'acide (méth)acrylique et/ou des esters de l'acide (méth)acrylique et en présence d'acide benzènesulfonique et/ou d'acide toluènesulfonique comme catalyseur.
